# EUROPEAN PATENT SPECIFICATION

(11) **EP 3 420 889 B1**
(45) Date of publication and mention of the grant of the patent: **15.01.2020**
(21) Application number: 17178484.6
(22) Date of filing: 28.06.2017
(51) Int. Cl.: A61B 3/113, A61H 5/00, G02B 27/01, G02B 27/00

(54) **APPARATUS FOR CHECKING THE EYESIGHT OF A USER**
VORRICHTUNG ZUR ÜBERPRÜFUNG DES SEHVERMÖGENS EINES BENUTZERS
APPAREIL PERMETTANT DE VÉRIFIER LA VISION D'UN UTILISATEUR

(43) Date of publication of application: 02.01.2019
(73) Proprietor: Vestel Elektronik Sanayi ve Ticaret A.S., 45030 Manisa (TR)
(72) Inventor: KUYUCU, Ömer Gökce, 45030 Manisa (TR)
(74) Representative: Flint, Adam

(56) References cited:
- US-A1- 2005 213 035
- US-A1- 2007 162 942
- US-A1- 2017 027 805
- US-B1- 7 354 156

## Description

### Technical Field

The present disclosure relates to an apparatus for checking the eyesight of a user.

### Background

There currently exist a number of devices for checking a person's eyesight, for example US 2017/027805 A1. A person's eyesight may be imperfect, for example, if the person suffers from a vison development disorder, such as amblyopia (also known as 'lazy eye'). Amblyopia occurs when (typically) one eye is unable to achieve normal visual acuity. Amblyopia is most prevalent in infants and children. Current techniques for monitoring amblyopia involve covering the stronger eye and forcing the weaker eye to work alone. While this technique has been shown to improve the visual acuity of the weaker eye it can also result in a weakening of the stronger eye. It can also be psychologically distressing for a child or infant that is having their stronger eye covered. Furthermore, known techniques for monitoring amblyopia often require the presence and expertise of an eye doctor.

### Summary

The invention is defined in the appended claims.

According to an aspect disclosed herein, there is provided an apparatus for checking the eyesight of a user, the apparatus comprising: a first lens which may be selectively closed to prevent light from reaching a first eye of a user; a second lens for displaying an image for viewing by a second eye of a user; a sensor configured to track a gaze direction of the user's second eye when an image is being displayed at the second lens; at least two audio output devices configured to output audio; and a processor configured to: receive an input from the sensor; determine, based on the received input, whether the user's second eye is directed at the displayed image; and in response to determining that the user's second eye is not directed at the displayed image, cause at least one of the audio output devices to output audio such that the output audio is located in space at a location that corresponds with the location at which the image is displayed at the second lens so as to draw the user's second eye to the displayed image.

The first lens, second lens, sensor and audio output devices may be provided in the form of glasses or spectacles or a headset or the like to be worn by a user. The processor may be provided integrally with the glasses or spectacles or headset or the like, or may be provided separately and in communication with the glasses or spectacles or headset or the like.

In an example, the processor is configured to cause data indicative of whether the user's second eye is determined as being directed at the displayed image to be stored in memory.

In an example, the apparatus is arranged such that the second lens displays an image comprising a plurality of objects, one of the plurality of objects being designated as a target object; and the processor is configured to determine whether the user's second eye is directed at the target object.

In an example, the apparatus is arranged such that the target object is displayed with a visual indication, the visual indication indicating that the user's second eye should be directed at the target object.

In an example, the apparatus is arranged such the second lens displays a moving image; and the processor is configured to determine whether the user's second eye is able to follow the moving image. The moving image may comprise a plurality of moving objects. The plurality of moving objects may move at the same or different speeds. The plurality of moving objects may also move in the same overall direction or in different directions.

In an example, the apparatus comprises a projector configured to project an image onto the second lens so as to provide an image for viewing by the second eye of the user.

In an example, the first lens is configured to selectively close by displaying no image or a black image.

In an example, the processor is configured to determine, based on the received input, whether the user's second eye is directed at the displayed image for a time period that exceeds a threshold time period.

In an example, the second lens is arranged to display different images at different times; and the processor is configured to: receive an input from the sensor for each displayed image; determine whether the user's second eye is directed at each of the displayed images; and cause data indicative of whether the user's second eye is determined to be directed at each of the displayed images to be stored in memory.

In an example, the apparatus comprises a transmitter operable to connect the apparatus to a separate client device by way of a wireless technology and wherein the transmitter is operable to transmit to the client device the data indicative of whether the user's second eye is determined to be directed at the displayed image. The processor may be configured to cause the data to be transmitted to the client device in a format that enables the data to be displayed at the client device.

In an example, both the first lens and the second lens is capable of displaying an image for viewing by a corresponding eye of the user and is selectively closable so as to prevent light from reaching a corresponding eye of the user. The apparatus may comprise a further sensor configured to track a gaze direction of the user's first eye when an image is being displayed at the first lens. The processor may be configured to receive an input from the further sensor and determine, based on the input received from the further sensor, whether the user's first eye is directed at the displayed image; and in response to determining that the user's first eye is not directed at the displayed image, cause at least one of the audio output devices to output audio such that the output audio is located in space at a location that corresponds with the location at which the image is displayed at the first lens so as to draw the user's first eye to the displayed image.

In an example, the apparatus comprises augmented reality glasses.

### Brief Description of the Drawings

To assist understanding of the present disclosure and to show how embodiments may be put into effect, reference is made by way of example to the accompanying drawings in which:
Figure 1 shows schematically an apparatus for checking the eyesight of a user;
Figure 2 shows schematically an aerial view of a user wearing an apparatus for checking the eyesight of a user;
Figure 3A schematically shows an example of an image displayed at a first location on a lens associated with a user's weaker eye;
Figure 3B schematically shows an example of an image displayed at a second location on a lens associated with a user's weaker eye;
Figure 3C schematically shows an example of an image displayed at a third location on a lens associated with a user's weaker eye;
Figure 4A schematically shows an example of an image comprising a plurality of objects for display at a lens associated with a user's weaker eye, a first of the plurality of objects being designated as a target object.
Figure 4B schematically shows an example of an image comprising a plurality of objects for display at a lens associated with a user's weaker eye, a second of the plurality of objects being designated as a target object.
Figure 4C schematically shows an example of an image comprising a plurality of objects for display at a lens associated with a user's weaker eye, where each of the plurality of objects have moved to a new location.
Figure 5 schematically shows an example of a process that might be implemented by a processor for checking the eyesight of a user.

### Detailed Description

Amblyopia, also known as 'lazy eye', is a vision development disorder in which the vision in one of the eyes is reduced because the eye and the brain are not working together properly. Generally, the earlier the treatment of amblyopia, the better the results will be.

In some cases, amblyopia may be corrected through conventional eye glasses. However, most treatments involve forcing a child or infant to user their weaker eye (by preventing them from using their stronger eye). This may be done, for example, by using an eye patch to cover the stronger eye for periods of time or by putting atropine in the stronger eye. One problem with this approach is that the child or infant may find the covering of their stronger eye to be a distressing experience. Moreover, the covering of the stronger eye may result in a weakening of the stronger eye at the expense of an improvement in the performance of the weaker eye.

More recent techniques for treating amblyopia involve the use of new technologies such as LCD screen glasses, where an opening and closing sequence can be used to ensure that the child is not prevented from using their stronger eye all of the time. One such system for treating amblyopia in this way is disclosed in US2016/0026009A1. However, current LCD screen glasses do not provide any interaction between the weaker eye and the glasses. For example, the LCD glasses cannot measure the performance of the child's weaker eye in real-time or track the overall performance of the eye over longer periods of time. Using known systems, it is not possible to determine whether visual acuity of the child's weaker is improving, at least not without having to consult an eye doctor.

Other techniques for treating amblyopia make use of virtual reality glasses to display visual effects to the child's weaker eye. However, these types of glasses are often not suitable for children and infants. Also, these types of glasses suffer from the same setbacks as the LCD screen glasses previously mentioned.

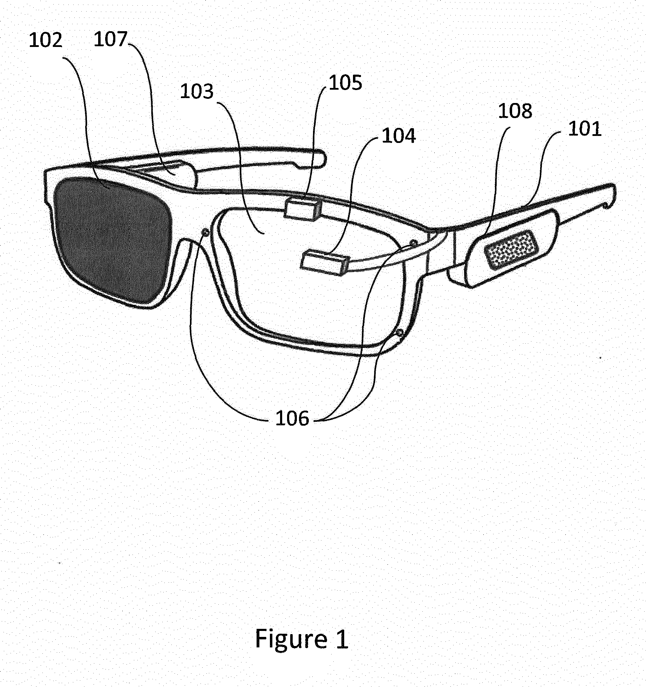

Figure 1 schematically illustrates an example of an apparatus 101 for checking the eyesight of a user in accordance with the present disclosure. In the example shown in Figure 1, the apparatus 101 is in the form of a pair of glasses or spectacles that can be worn by the user. For example, the glasses may include a frame for mounting the glasses to the head of the user. The frame may include a first and second support for balancing the glasses on the ears of the user. The frame may also include a bridge for balancing the glasses on the nose of the user.

In some embodiments, the glasses may correspond to a pair of augmented reality or virtual reality glasses. Here, the term augmented reality glasses refers to glasses that enable a computer-generated image to be superimposed over a user's view of the real world. The combination of the computer-generated image and the view of the real world may be referred to as an augmented reality image. In other embodiments, the apparatus 101 comprises a pair of virtual reality glasses where the user is only able to view a virtual world, as opposed to a combination of the real world and virtual objects superimposed over the user's view of the real world.

As can be seen in Figure 1, the apparatus 101 comprises a first lens 102 that is operable to be selectively closed so as to prevent light from reaching a first eye of the user. In Figure 1, the first lens 102 is shown as being closed by way of darker patterning. The first lens 102 may be selectively closed by causing the lens to display no image or a black image. For example, the first lens 102 may comprise an LCD screen and the LCD screen may be controlled so as to display no image. Alternatively, a separate shutter (not shown) may be attached to the front of the first lens 102 and the first lens 102 may be selectively closed by closing the shutter or placing the shutter over the front of the first lens 102.

The apparatus 101 further comprises a second lens 103 that is operable to display an image for viewing by a second eye of a user. The second lens 103 may be associated with a projector 104, such as for example a micro-projector or pico-projector, for generating an image at the second lens 103. The projector 104 is configured to project an image onto the second lens 103 for viewing by the user. The projector 104 may be configured to project an image that only occupies a portion of the second lens 103, such that the user is still able to see through the portions that are not being used to display the image. In embodiments where the apparatus 101 comprises a pair of glasses, the projector 104 may be embedded within the frame or attached to the front of the frame. In other examples, the second lens 103 may be arranged to display the image directly.

In some examples, both the first lens 102 and the second lens 103 may be operable to be selectively closed and selectively operable to display an image as described herein so that the user's left eye or right eye can be checked at choice.

In some examples, the first lens 102 and second lens 103 may be contiguous in that the two lenses are not partitioned from one another by e.g. the frame. Nevertheless, for present purposes, one side may be regarded as providing a first lens 102 for one eye and a second lens 103 for the other eye. A controller (not shown) may be programmed with information relating to the sides of the lens that correspond to the field of view of the user's first and second eyes. The controller may use this information to ensure that an image is only displayed at the side of the lens that is in front of the user's weaker eye.

The second lens 103 may also be associated with a sensor 105 that is configured to detect a gaze-direction of the user's second eye. For example, the sensor 105 may employ known infra-red eye tracking to track any movement of the pupil of the user's second eye. The sensor 105 in this example is provided on the apparatus 101 in the region of the second lens 103. The sensor 105 may be configured to provide a detected gaze-direction of the user's second eye as an input to a processor. The processor may be configured to determine whether the detected gaze-direction of the user's second eye corresponds to a gaze-direction that would be expected if the second eye were directed at an image displayed at the second lens 103. In one example of an embodiment, the sensor 105 may be attached to the outward facing part of the frame enclosing the second lens 103 (i.e. in front of the second lens 103).

The sensor 105 may be configured to track the gaze-direction of the user's second eye over a given time period. For example, the sensor 105 may periodically provide measurements of the gaze-direction (or data indicative thereof) to the processor. The processor may be configured to determine, based on the received measurements, whether the user's second eye is directed at the displayed image, and if it is, a duration for which the user's second eye is directed at the displayed image.

As can also be seen in Figure 1, the apparatus 101 comprises at least two audio output devices 106 for outputting audio to a user. The at least two audio output devices 106 may comprise speakers, which may for example be piezoelectric speakers or buzzers.

The at least two audio output devices 106 are positioned at different locations on the apparatus 101. For example, where the apparatus 101 is in the form of a pair of glasses or the like, the at least two audio output devices 106 may be attached to, or embedded within different parts of the frame. It will be appreciated that the apparatus 101 may comprise any number of audio output devices 106 so long as the number is equal to or greater than two.

In one particular embodiment, the apparatus 101 comprises three audio output devices 106. This is shown in Figure 1, where it can be seen that three speakers 106 are located at different positions around the second lens 103. In this example, the first speaker 106 is located at a bridge region of the glasses and the second and third speakers 106 are located at the upper right hand and lower right hand of the frame respectively. In other examples, audio output devices 106 may be located at the leftmost and rightmost sides of the apparatus 101, optionally with one or more audio output devices 106 located between the extreme left side and right side audio output devices 106. Each audio output device 106 may be configured to output, for example, a sound effect that relates to the image displayed at the second lens 103.

The processor is configured to cause at least one of the audio output devices 106 to output audio in response to determining that the user's second eye is not directed at the image displayed at the second lens 103. This may be, for example, so as to draw the user's second eye to or towards the image displayed at the second lens 103. The processor may be configured to identify which of the at least two audio output devices 106 is located closest to the displayed image (or most able to influence the location at which the image is displayed) and cause the corresponding audio output device 106 to output audio. This identification may be based on, for example, pixel information relating to where the image is displayed at the second lens 103. This information may be received, for example, at the processor. In some examples, the processor may be configured cause both of the at least two audio output devices 106 to output audio. This may be so as to create the impression of a single sound source originating from a location in space that corresponds to the location at which the image is being displayed at the second lens 103 (i.e. via a stereophonic effect). In an alternative, the sound source may be located so as to be beyond the location at which the image is being displayed at the second lens 103. For example, if the image is being displayed at the second lens 103 at a location that is somewhat to the left of centre, then audio may be played back through the audio output devices 106 so as to be located further to the left of the image.

By creating an audio output that appears to originate from (or beyond) the location at which the image is displayed at the second lens 103, the child or infant's weaker eye will, in general, be drawn to or at least towards the image displayed at the second lens 103. This is advantageous over known systems which provide only a visual stimulus for guiding the child or infant's eye to a desired target image. The apparatus 101 of the present disclosure provides both a visual stimulus and an aural stimulus for this purpose. The user's weaker eye may be drawn to the displayed image where it would otherwise not have been (i.e. for the visual stimulus only). In this way, the user's weaker eye can be better trained to look at different images, which in turn may lead to an improvement in the eyesight of the user's weaker eye.

In examples, the audio output may be such as to be particularly engaging for a child or infant to interact with. For example, the displayed image may correspond to a cartoon cow, and the processor may cause a 'moo' sound effect to be output by one or more of the audio output devices 106. In some embodiments, the sound effect may correspond to a person's speech. For example, the displayed image may include a cartoon character, and the processor may be configured to cause the cartoon character to say 'hey! I'm over here!' in response to determining that the user's second eye 103 is not directed at the displayed image. In this manner, the user's interaction with the apparatus 101 may be experienced in a game-like manner. This may have the overall of effect of encouraging the user to use the apparatus, which may in turn lead to an improvement in the eyesight of the user's weaker eye.

In some embodiments, the processor may also be configured to cause at least one of the audio output devices 106 to output audio in response to determining that the user's second eye is directed at the displayed image. Again, the audio output may be located in space such that the location corresponds with the location at which the image is displayed at the second lens. The processor may be configured to cause a different sound effect to be output, compared to the sound effect that would be output in response to determining that the user's second eye is not directed at the displayed image. This may act so as to provide positive reinforcement for the child or infant who is using the apparatus. For example, the cartoon character described previously may be caused to say 'You found me!', in response to the processor determining that the user's eye is directed at the displayed image.

As can also be seen in Figure 1, the apparatus 101 may further comprise a power source 107, such as a battery which may be rechargeable, for powering one or more components that make up the apparatus 101. The power source 107 may, for example, provide a supply of power to one or more of the projector 104, sensor 105, processor and one or more audio output devices 106 described previously.

The apparatus 101 may also comprise an electronic assembly 108 in which the processor and memory is contained. That is, in this example, the processor is provided integrally with the glasses spectacles or headset or the like. In other examples, the processor may be provided separately and be in wired or wireless communication with the glasses or spectacles or headset or the like. The processor may perform any of the functions described in accordance with the present disclosure. The memory may be configured to store data indicative of whether the user's second eye is determined as being directed at the displayed image. This data may be considered indicative of the visual acuity of the user's second eye.

The processor may be associated with a clock or timer. The clock or timer may be used by the processor to ensure that the data stored in memory is stored in association with a time value. For example, the time value may include a date, month, year on which it was determined whether the user's second eye was able to look at the displayed image. By saving data in association with a respective time value, a historical record of the visual acuity of the user's second eye 204 can be built up in memory.

The electronic assembly 108 may also include a transmitter (not shown) operable to connect the apparatus 101 to a separate client device by way of a wireless technology. The client device may include, for example, a smartphone, tablet, PC, etc. The wireless technology may include for example, Bluetooth or WiFi. The transmitter may be operable to transmit data indicative of whether the user's second eye is determined as being directed at the displayed image to the client device. This may be done, for example, in response to receiving a request for the data from the client device. The request may be received at a receiver associated with the apparatus 101 (not shown). Alternatively, the transmission may performed automatically, in response to the processor determining whether the user's second eye is directed at the displayed image. The data downloaded from the apparatus 101 may be displayed at the client device in such a manner that the user or a guardian or parent, etc. of the user can easily determine whether the performance of the user's weaker eye is improving.

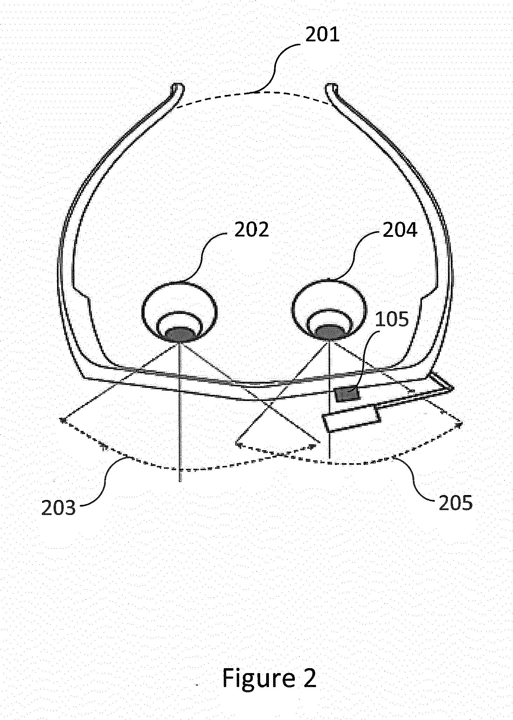

Figure 2 shows schematically a plan view of a user 201 wearing an example of an apparatus 101 according to the present disclosure. As can be seen in Figure 2, the user's first eye 202 is the stronger eye with a respective field of view 203. The user's second eye 204 is the weaker eye with a respective field of view 205. The sensor 105 is positioned so as to enable the gaze-direction of the second eye 204 to be determined.

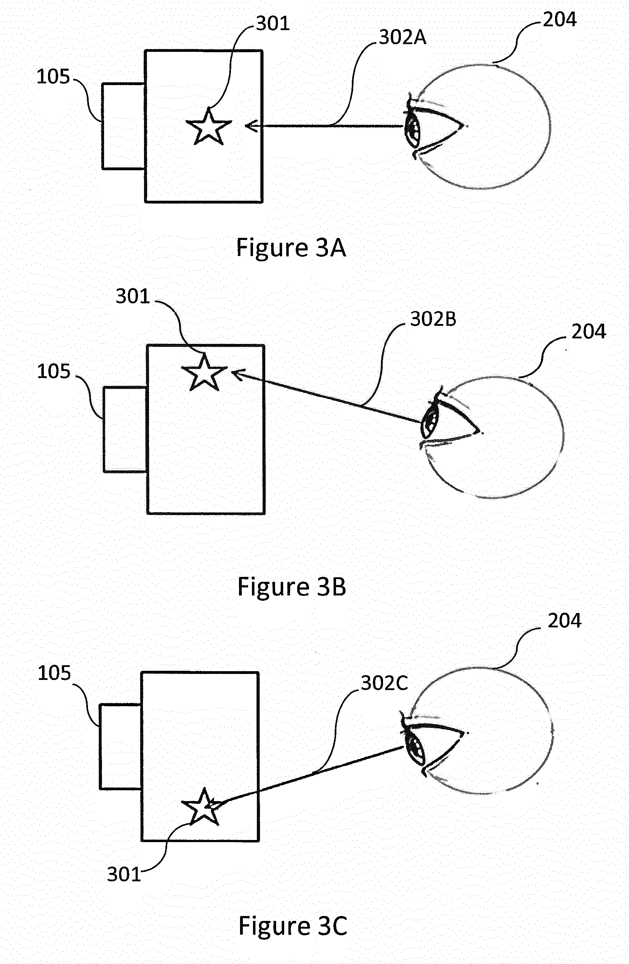

Figure 3A schematically shows an example of an apparatus 101 according to the present disclosure in which the second lens 103 is displaying an image 301. The gaze-direction of the user's second eye 204 is indicated with vector 302A. The sensor 105 is configured to determine the direction of vector 302A. In the specific example shown in Figure 3A, the image 301 comprises a star.

In some examples, the image 301 displayed at the second lens 103 may only occupy only a portion of the second lens 103 such that the user's view of the real world is only partially inhibited by the displayed image 301. This image may be considered as an object forming part of an augmented reality image. The second lens 103 may be operable to display an object of any shape or colour.

The image displayed at the second lens 103 may comprise a static image. The display of static images at the second lens 103 may be considered as a first mode of operation of the apparatus 101.

The processor may cause an action to be performed in response to detecting that the user's second eye 204 is directed at the displayed image 301. For example, the processor may modify the image 301 or cause a new image to be displayed. Modifying the displayed image 301 may involve causing the displayed image 301 to be displayed at a different location on the second lens 103 or animating the displayed image 301 such that it moves to a new location on the second lens 103.

In some embodiments, the processor may also be configured to modify the displayed image 301 in response to determining that the user's second eye 204 is not directed at the displayed image 301. For example, the processor may cause the displayed image 301 to change colour. This may involve for example displaying the image in red instead of green. This modification of the displayed image can be combined with the audio that is output by at least one of the audio output devices 106, in response to the processor determining that the user's second eye 204 is not directed at the displayed image. In this way, the user is provided with both visual and aural feedback.

The second lens 103 may be operable to display the same image 301 at a new position on the second lens 103. This is shown schematically in Figure 3B, which shows the same image 301 displayed in Figure 3A, but at a new location on the second lens 103. The processor may cause the same image 301 to be displayed at a new location in response to determining that the user is able to direct their gaze towards the image 301 displayed at the previous location. In the example illustrated in Figure 3B, the user's weaker eye 204 is shown with vector 302 having a direction that corresponds to the new location at which the image 301 is displayed at the second lens 103.

Additionally or alternatively, the processor may cause a different image to be displayed at the new position on the second lens 103 in response to determining that the user's second eye 204 is able to focus on the image 301 displayed at the previous location.

The processor may be configured to determine whether the user's second eye 204 is directed at the image displayed at the new position for a time period that exceeds a threshold time period. The threshold time period may be stored in memory (i.e. it may be predetermined). In some embodiments, the memory is configured to store a plurality of different threshold time periods in association with different images for display. For example, the threshold time period associated with a current image being displayed at the second lens 103 may be longer than a threshold time period associated with an image previously displayed at the second lens 103. This may act so as to gradually train the user's second eye.

In response to detecting that the user's second eye 204 is directed at the image displayed at the new position for a sufficiently long time, the second lens 103 may be operable to display the same image at another new location on the second lens 103. An example of this is shown in Figure 3C, which shows a third location on the second lens 103 at which the displayed image 301 might be displayed. In the example shown in Figure 3C, the user's weaker eye 204 is shown with vector 406C directed at the image 301 displayed at another new location on the second lens 103.

In some examples, the new location at which the image is displayed may be random. In other examples of embodiments, if the processor determines that the user 201 is unable to focus on the image displayed at the current location, the image may be displayed at a new location that is closer to a location for which the user's second eye 204 is recorded as having been able to look at previously. In some embodiments, displaying the image at a new location may comprise causing the current image to be removed from the display and displaying the image at the new location.

In some examples, the second lens 103 may be operable to cause the image displayed at a first location on the second lens 103 to be moved to a new location on the second lens 103 by way of animation, i.e. as a moving image. The processor may be configured to determine whether the user's second eye 204 is able to follow the displayed image as it moves from its initial position to a new position on the second lens 103. If the processor determines that the user's second eye 204 is able to follow moving image, the processor may cause the second lens 103 to display the image at a different location. If the processor determines that the user's second eye 204 is not able to follow the moving image, the processor causes the audio output devices 106 to output audio at a location that changes with the changing location of the moving image displayed at the second lens 103. In preferred embodiments, this is achieved using two or more audio output devices (i.e. by generating a stereophonic effect).

Movement of the image displayed at the second lens 103 may be thought of as a second mode. The second mode may be made available, for example, once it has been determined that the user's second eye 204 is able to sufficiently focus on static images displayed at different locations of the second lens 103.

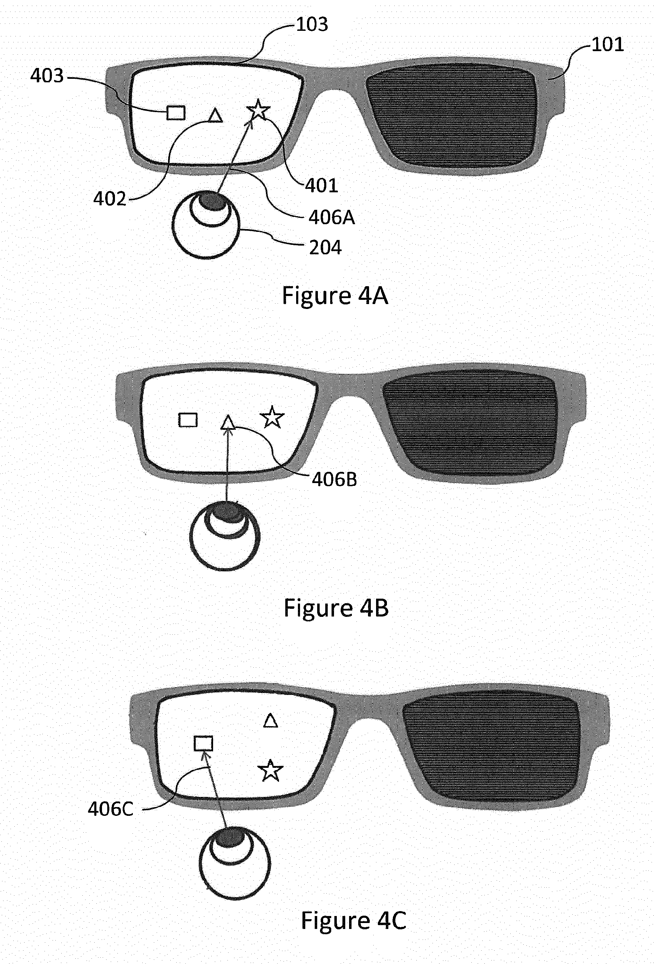

Figures 4A-4C schematically show an example of an apparatus 101 in which the second lens 103 is displaying an image comprising a plurality of different objects.

As can be seen in the example shown in Figure 4A, the first object 401 comprises a star, the second object 402 comprises a triangle and the third object 403 comprises a rectangle. It will be appreciated that the second lens 103 may be operable to display any number of objects. Each object occupies only a portion of the second lens 103. The gaze-direction of the user's second eye 204 is indicated with vector 406A.

The display of a plurality of different objects at the second lens 103 may be considered as a third mode. In the third mode, the user may be required to direct their gaze at each of the displayed objects sequentially. For example, in the example shown in Figures 4A-4C, the user may be required to direct the gaze of their second eye to the star first, the triangle second and the rectangle third. In the third mode, each of the plurality of images may be static images.

In the third mode, one of the objects may be designated as a target object. The target object corresponds to an object that the user's second eye 204 should be looking at. The target object may be displayed with a visual indication. For example, the target object may be displayed in a different colour and/or with a bolder outline. Additionally or alternatively, the target object may be animated. For example, the star shown in Figure 4A may be animated with a pulsing effect such that it remains stationary but is displayed with a varying size.

In the third mode, the processor is configured to determine whether the user's second eye 204 is directed at the target object. If the processor determines that the user's second eye 204 is not directed at the target object, the processor causes at least one audio output device 106 to output audio at a location corresponding to the location at which the target object is displayed at the second lens 103. That is, the output audio is localized to the target object. As mentioned previously, such an audio output may be generated by combining the audio output by at least two audio output devices 106 (i.e. stereophonically). This may have the effect of drawing the user's second eye 204 towards the target object.

If the processor determines that the user's second eye 204 is directed at the target object, the processor may cause a different one of the displayed objects to be selected as a new target object. The processor may then cause the new target object to be displayed differently instead of the object that was previously used as the target object. The previous target object may then be displayed in its original manner (e.g. with the colour, fill or outline that it had prior to its selection as the target object). An example of this is shown schematically in Figure 4B, where it can be seen that object 402 (the triangle) is the new target object. In Figure 4B, a user's second eye 204 that is directed at the new target object is shown as having a gaze-direction vector 406B.

In some examples, the processor may be configured to cause a change in the appearance of the target object, in response to determining that the user's second eye is directed at the target object. This may add another level of visual feedback for the user.

Figure 4C schematically shows an example in which each of the plurality of different objects has been moved to new locations on the second lens 103. This may be caused, for example, in response to the processor determining that the user's second eye 204 is able to look at a previous target object. In Figure 4C, a user's second eye 204 that is directed at the new target object is shown as having a gaze-direction vector 406C.

In some examples, the second lens 103 may be arranged to display each of the objects as moving objects. The plurality of different objects may move independently of each other (e.g. in different directions and/or at different speeds) or may move in a related manner (e.g. collectively in the same overall direction and/or at the same speed).

The processor may be configured to determine whether the user's second eye 204 is able to follow one of the moving objects. In other words, the target object may be a moving object. If the processor determines that the user's second eye is not able to follow the moving target object, the processor causes the audio output devices 106 to output audio in such a manner that the audio is output at a location that changes with the changing location of the target object displayed at the second lens.

The moving target object may correspond to an object that was previously displayed as a static object. The processor may be configured to determine whether the user's second eye is able to follow the moving target object for a time period that exceeds a threshold time period. Requiring that the user's second eye follows a moving target object may be considered as a fourth mode of operation.

In response to detecting that the user's second eye 204 is able to follow the moving target object for a sufficiently long period of time, the processor may select a different one of the plurality of objects as a target object. The processor may then determine whether the user's second eye 204 is able to follow the new target object for a sufficiently long period of time.

In some examples of embodiments, in response to detecting that the user's second eye 204 is able to follow a moving target object for a sufficiently long period of time, the processor causes the speed and/or direction of the current target object, or the next target object, to change.

While the first, second, third and fourth modes have been described separately and in a successive order, it will be appreciated that in general the user may experience these modes in any given order. Furthermore, in some embodiments, the performance of the user's second eye during for example the first mode may not preclude the user's ability to experience any of the subsequent modes. In other embodiments, each mode may be associated with an associated difficulty, and later modes may not be made available to the user until the user's second eye 204 has met a minimum performance requirement in the earlier modes.

It will be appreciated that the image displayed at the second lens 103 need not be limited to simple shapes such as those shown in Figures 3A-3C and Figures 4A-4C. In some examples of embodiments, the image displayed at the second lens 103 may become increasingly complex as the processor determines that the user's second eye is able to gaze at an increasing number of images.

In some examples, the user may be able to control the images (or indeed objects making up the images) that are displayed at the second lens 103. For example, the apparatus 101 may be associated with a client application running at a separate client device that is able to connect to the apparatus 101 via the Bluetooth or Wi-Fi transmitter associated with the apparatus 101. The client application may enable the user or for example a parent or guardian of the user to select from a menu a theme relating to the images that will be displayed at the client device. The client application may provide a plurality of different themes, each being deemed appropriate for users of different ages. Additionally or alternatively, the client application may enable for example the parent or guardian to customise the images that are displayed to the user.

By allowing the user (or their parent or guardian) to exert some level of control over the images that are displayed at the second lens 103, it can be ensured that the user's experience of using the apparatus 101 is a more positive one. This increases the likelihood that the user will keep using the apparatus 101, which in turn may lead to an eventual improvement in the performance of the user's weaker eye 204.

The client application may also enable the user 201, or more preferably their parent or guardian, to use a recording of their speech as a source of audio to be output by the audio output devices 106. This may provide another level of comfort for the child or infant that is unfamiliar with the apparatus and potentially anxious about using it.

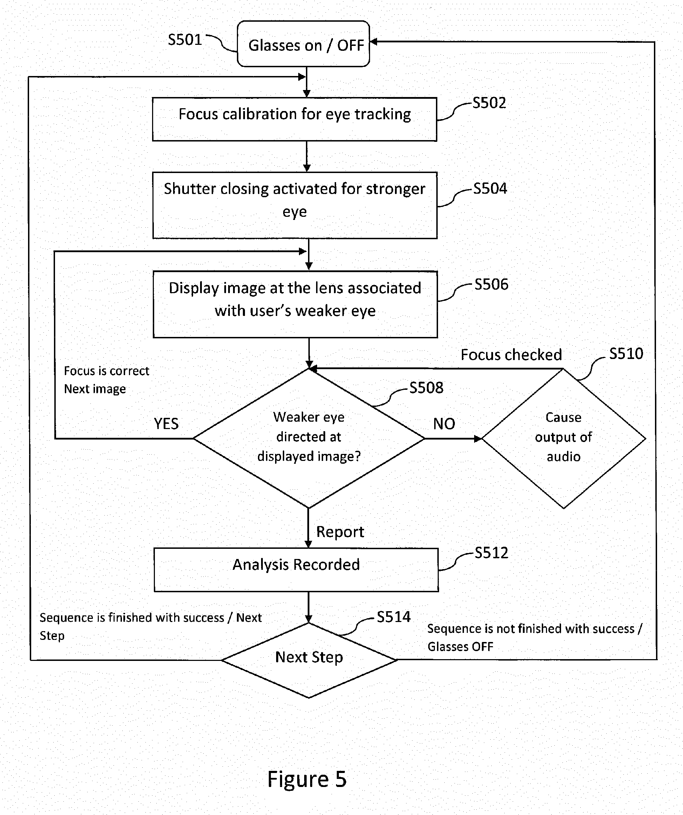

Figure 5 shows an example of a process that may be implemented when the apparatus 101 according to the present disclosure is in use.

At an initial step, step 501, the apparatus 101 is switched on. At step 502, the sensor 105 is calibrated to one or both of the user's eyes. This involves, for example, detecting the pupil and/or iris (through e.g. image analysis) of one or both of the user's eyes so that movement of one or both of the user's eye can be tracked. In some embodiments, the user may provide an indication, by way of e.g. a user input, indicating which of their eyes is the weaker eye and the calibration process may be limited to that eye only.

At step S504, the lens 102 associated with the user's stronger eye 202 is selectively closed so as to prevent light from reaching the user's stronger eye 202. The lens 102 associated with the user's stronger eye 202 may cycle between periods of being selectively closed and selectively open. The user's stronger 202 eye may be identified manually (i.e. by the user via a user input) or as part of the calibration step S502. Selectively closing the lens 102 associated with the user's stronger eye 202 may be performed as described previously.

At step S506, an image is displayed at the lens associated with the user's weaker eye 204. The image displayed at this lens may correspond to any of the images described previously.

At step S508 the processor determines whether the user's weaker eye 204 is directed at the image displayed at the lens associated with the user's weaker eye.

If the processor determines that the user's weaker eye 204 is directed at the displayed image, or that the user's weaker eye 204 is not directed at displayed image for a sufficient period of time, the processor causes at least one of the audio output devices 106 to output audio. This is shown at step S510. As described previously, the apparatus 101 comprises at least two audio output devices 106 and the processor is configured to cause at least one of the audio output devices 106 to output audio such that the output audio is located in space at a location in space that corresponds with the location at which the image is displayed at the second lens 103.

In response to causing at least one audio output device 103 to output audio, the processor may then determine whether the user's weaker eye 204 is now directed at the displayed image. This step may be iterated any number of times until the processor determines that the user's weaker eye 204 is able to sufficiently focus on the displayed image.

If, following step S508, the processor determines that the user's weaker eye 204 is directed at the displayed image, or that the user's weaker eye 204 is directed at the displayed image for a sufficient period of time, the processor may cause the lens to display a new image and/or to modify the image that is displayed at the lens associated with the weaker eye 204. As described previously, this may involve, for example, displaying a moving image and/or selecting a different object as a target object.

At step S512, an indication of whether the user's weaker eye 204 is determined as being directed at the displayed image is recorded in memory. The indication stored in memory may indicate a positive or negative result (i.e. an indication is stored in memory for both outcomes, the indication providing an indication of the outcome).

The displayed image may form one image in a sequence of images that are to be displayed to the user's weaker eye 204. In this embodiment, a measurement indicative of the user's ability to look at each of the images in the sequence may be stored in memory. In additional or alternative embodiments, a measurement indicative of the user's overall ability to look at the images in the sequence may be stored in memory.

At step S514, the apparatus 101 may be switched off in response to the user 201 having viewed all of the images in the sequence of images. In preferred embodiments, the apparatus may be switched off in response to determining that a user 201 is unable to look at an image, or at a sufficient number of images in the sequence of images. Switching off the apparatus 101 may result in both of the lenses (102, 103) allowing light to reach the user's eyes.

If the user 201 is determined as being able to look at a sufficient number of images in the sequence of images, the apparatus may repeat the calibration step (S502), in preparation for the new sequence of images that are to be displayed to the user. This is shown as an alternative option to step S514. The process may then be repeated for each new image in the new sequence of images displayed at the second lens 103.

As described previously, the indication of whether the user's weaker eye 204 is able to look at the displayed image may be stored in association with a time, such as the time at which it was determined whether the user's weaker eye 204 was able to look at the displayed image. The time may include the time of the measurement, as well as a day, month and year at which the measurement was taken. Each time the process illustrated in Figure 5 is repeated, the resulting indication may be stored in memory. This enables a historical record of the performance of the user's weaker eye 204 to be built up.

The historical record of the weaker eye's performance may be accessible to for example a parent or guardian of the user, for example by the parent or guardian connecting their client device to the apparatus 101 (using e.g. Bluetooth or WiFi as described previously) and downloading the historical data to their device. The client application at the client device may cause the historical data to be displayed at the client device (or a display device associated therewith) in a format from which the performance of the user's weaker eye 204 can easily be understood. For example, in one embodiment the historical data is displayed as a graph, with time indicated on the x-axis, and a score indicative of the weaker eye's performance on the y-axis. A score for which the weaker eye 204 can be considered as performing normally may also be displayed as part of the historical data. Thus the parent or guardian of the user can determine for themselves how close or far the user's weaker eye 204 is from performing normally.

By displaying the historical data in a simple and intuitive manner, the parent or guardian of the user can determine whether the visual acuity of the user's weaker eye 204 is improving, without needing to consult a professional eye doctor. The historical data may also enable the parent or guardian to re-configure the apparatus 101. For example, a parent might select a different theme of images to be displayed at the second lens 103 if it appears that the child's weaker eye 204 is not responding to the current theme of images being displayed. The different theme of images may be associated with different sound effects (i.e. relating to the theme of images).

It will be understood that the processor or processing system or circuitry referred to herein may in practice be provided by a single chip or integrated circuit or plural chips or integrated circuits, optionally provided as a chipset, an application-specific integrated circuit (ASIC), field-programmable gate array (FPGA), digital signal processor (DSP), graphics processing units (GPUs), etc. The chip or chips may comprise circuitry (as well as possibly firmware) for embodying at least one or more of a data processor or processors and a digital signal processor or processors, which are configurable so as to operate in accordance with the exemplary embodiments. In this regard, the exemplary embodiments may be implemented at least in part by computer software stored in (non-transitory) memory and executable by the processor, or by hardware, or by a combination of tangibly stored software and hardware (and tangibly stored firmware).

Reference is made herein to data storage for storing data. This may be provided by a single device or by plural devices. Suitable devices include for example a hard disk and non-volatile semiconductor memory.

The examples described herein are to be understood as illustrative examples of embodiments of the invention. Further embodiments and examples are envisaged. Any feature described in relation to any one example or embodiment may be used alone or in combination with other features. In addition, any feature described in relation to any one example or embodiment may also be used in combination with one or more features of any other of the examples or embodiments, or any combination of any other of the examples or embodiments. The scope of the invention is defined in the claims.

## Claims

1. Apparatus for checking the eyesight of a user (201), the apparatus comprising:
a first lens (102) which may be selectively closed to prevent light from reaching a first eye of a user (202);
a second lens (103) for displaying an image for viewing by a second eye (204) of a user (201);
a sensor (105) configured to track a gaze direction of the user's second eye (204) when an image is being displayed at the second lens (103); and
at least two audio output devices (106) configured to output audio; and
a processor configured to:
receive an input from the sensor (105);
determine, based on the received input, whether the user's second eye (204) is directed at the displayed image; and
in response to determining that the user's second eye (204) is not directed at the displayed image, cause at least one of the audio output devices (106) to output audio
**characterised in that**
the audio is output such that the output audio is located in space at a location that corresponds with the location at which the image is displayed at the second lens (103) so as to draw the user's second eye to the displayed image.

2. An apparatus according to claim 1, wherein the processor is configured to cause data indicative of whether the user's second eye (204) is determined as being directed at the displayed image to be stored in memory.

3. An apparatus according to claim 1 or claim 2, arranged such that the second lens (103) displays an image comprising a plurality of objects, one of the plurality of objects being designated as a target object; and
the processor is configured to determine whether the user's second eye (204) is directed at the target object.

4. An apparatus according to claim 3, arranged such that the target object is displayed with a visual indication, the visual indication indicating that the user's second eye (204) should be directed at the target object.

5. An apparatus according to any of claims 1 to 4, arranged such that the second lens (103) displays a moving image; and
the processor is configured to determine whether the user's second eye (204) is able to follow the moving image.

6. An apparatus according to any of claims 1 to 5, comprising a projector configured to project an image onto the second lens (103) so as to provide an image for viewing by the second eye (204) of the user.

7. An apparatus according to any of claims 1 to 6, wherein the first lens (102) is configured to selectively close by displaying no image or a black image.

8. An apparatus according to any of claims 1 to 7, wherein the processor is configured to determine, based on the received input, whether the user's second eye (204) is directed at the displayed image for a time period that exceeds a threshold time period.

9. An apparatus according to any of claims 1 to 8, wherein:
the second lens (103) is arranged to display different images at different times; and
the processor is configured to:
receive an input from the sensor (105) for each displayed image;
determine whether the user's second eye (204) is directed at each of the displayed images; and
cause data indicative of whether the user's second eye (204) is determined to be directed at each of the displayed images to be stored in memory.

10. An apparatus according to any of claims 1 to 9, comprising a transmitter operable to connect the apparatus to a separate client device by way of a wireless technology and wherein the transmitter is operable to transmit to the client device the data indicative of whether the user's second eye is determined to directed at the displayed image.

11. An apparatus according to any of claims 1 to 9, wherein both the first lens and the second lens (103) is capable of displaying an image for viewing by a corresponding eye of the user and is selectively closable so as to prevent light from reaching a corresponding eye of the user.

## Patentansprüche

1. Vorrichtung zur Überprüfung des Sehvermögens eines Benutzers (201), wobei die Vorrichtung umfasst:
Eine erste Linse (102), die selektiv geschlossen werden kann, um zu verhindern, dass Licht ein erstes Auge eines Benutzers (202) erreicht;
eine zweite Linse (103) zur Anzeige eines Bildes zur Betrachtung durch ein zweites Auge (204) eines Benutzers (201);
einen Sensor (105), der konfiguriert ist, eine Blickrichtung des zweiten Auges (204) des Benutzers zu verfolgen, wenn ein Bild an der zweiten Linse (103) angezeigt wird; und
zumindest zwei Audio-Ausgabegeräte (106), die konfiguriert sind, Audio auszugeben; und
einen Prozessor, der konfiguriert ist, Folgendes zu tun:
Eine Eingabe vom Sensor (105) zu empfangen;
Ermitteln, auf Basis der empfangenen Eingabe, ob das zweite Auge (204) des Benutzers auf das angezeigte Bild gerichtet ist; und
als Antwort auf das Ermitteln, dass das zweite Auge (204) des Benutzers nicht auf das angezeigte Bild gerichtet ist, zu bewirken, dass zumindest eins der Audio-Ausgabegeräte (106) Audio ausgibt, **dadurch gekennzeichnet, dass** das Audio derartig ausgegeben wird, dass sich das ausgegebene Audio im Raum an einer Stelle befindet, die der Stelle entspricht, an der das Bild an der zweiten Linse (103) angezeigt wird, um das zweite Auge des Benutzers zum angezeigten Bild zu lenken.

2. Vorrichtung nach Anspruch 1, wobei der Prozessor konfiguriert ist, zu bewirken, dass Daten, die darauf hindeuten, ob das zweite Auge (204) des Benutzers bestimmt ist, als auf das angezeigte Bild gerichtet zu sein, im Speicher gespeichert werden sollen.

3. Vorrichtung nach Anspruch 1 oder Anspruch 2, die derartig eingerichtet ist, dass die zweite Linse (103) ein Bild anzeigt, das eine Vielzahl von Objekten umfasst, wobei eins der Vielzahl von Objekten als ein Zielobjekt designiert ist; und
der Prozessor konfiguriert ist, zu ermitteln, ob das zweite Auge (204) des Benutzers auf das Zielobjekt gerichtet ist.

4. Vorrichtung nach Anspruch 3, die derartig eingerichtet ist, dass das Zielobjekt mit einer visuellen Anzeige angezeigt wird, wobei die visuelle Anzeige anzeigt, dass das zweite Auge (204) auf das Zielobjekt gerichtet werden sollte.

5. Vorrichtung nach einem der Ansprüche 1 bis 4, die derartig eingerichtet ist, dass die zweite Linse (103) ein sich bewegendes Bild anzeigt; und
der Prozessor konfiguriert ist, zu ermitteln, ob das zweite Auge (204) des Benutzers fähig ist, dem sich bewegenden Bild zu folgen.

6. Vorrichtung nach einem der Ansprüche 1 bis 5, die einen Projektor umfasst, der konfiguriert ist, ein Bild auf die zweite Linse (103) zu projizieren, um ein Bild bereitzustellen, das sich mit dem zweiten Auge (204) des Benutzers betrachten lässt.

7. Vorrichtung nach einem der Ansprüche 1 bis 6, wobei die erste Linse (102) konfiguriert ist, sich selektiv zu schließen, indem kein Bild oder ein schwarzes Bild angezeigt wird.

8. Vorrichtung nach einem der Ansprüche 1 bis 7, wobei der Prozessor konfiguriert ist, auf Basis der empfangenen Eingabe, zu ermitteln, ob das zweite Auge (204) des Benutzers auf das angezeigte Bild für einen Zeitraum gerichtet ist, der einen Schwellenwertzeitraum überschreitet.

9. Vorrichtung nach einem der Ansprüche 1 bis 8, wobei:
Die zweite Linse (103) eingerichtet ist, verschiedene Bilder mit verschiedenen Zeiten anzuzeigen; und
der Prozessor konfiguriert ist, Folgendes zu tun:
Empfangen einer Eingabe vom Sensor (105) für jedes angezeigte Bild;
Ermitteln, ob das zweite Auge (204) des Benutzers auf jedes der angezeigten Bilder gerichtet ist; und
Bewirken, dass Daten, die darauf hindeuten, ob das zweite Auge (204) des Benutzers bestimmt ist, auf jedes der angezeigten Bilder gerichtet zu sein, im Speicher gespeichert werden sollen.

10. Vorrichtung nach einem der Ansprüche 1 bis 9, die einen Sender umfasst, der betriebsfähig ist, die Vorrichtung mit einem separaten Client-Gerät mittels einer drahtlosen Technologie zu verbinden und wobei der Sender betriebsfähig ist, dem Client-Gerät die Daten zu senden, die darauf hindeuten, ob das zweite Auge des Benutzers bestimmt ist, auf das angezeigte Bild gerichtet zu sein.

11. Vorrichtung nach einem der Ansprüche 1 bis 9, wobei sowohl die erste Linse als auch die zweite Linse (103) fähig ist, ein Bild zum Betrachten mit einem entsprechenden Auge des Benutzers anzuzeigen, und selektiv schließbar ist, um zu verhindern, dass Licht ein entsprechendes Auge des Benutzers erreicht.

## Revendications

1. Appareil servant à vérifier la vision d'un utilisateur (201), l'appareil comportant :
un premier verre (102) qui peut être fermé de manière sélective pour empêcher la lumière d'atteindre un premier œil (202) d'un utilisateur ;
un deuxième verre (103) servant à afficher une image à des fins de visualisation par un deuxième œil (204) d'un utilisateur (201) ;
un capteur (105) configuré pour suivre une direction du regard du deuxième œil (204) de l'utilisateur quand une image est affichée au niveau du deuxième verre (103) ; et
au moins deux dispositifs de sortie audio (106) configurés pour émettre en sortie un signal audio ; et
un processeur configuré pour :
recevoir une entrée en provenance du capteur (105) ;
déterminer, en fonction de l'entrée reçue, si le deuxième œil (204) de l'utilisateur est dirigé au niveau de l'image affichée ; et
en réponse à la détermination comme quoi le deuxième œil (204) de l'utilisateur n'est pas dirigé au niveau de l'image affichée, amener au moins l'un des dispositifs de sortie audio (106) à émettre en sortie un signal audio,
**caractérisé en ce que**
le signal audio est émis en sortie de telle sorte que le signal audio émis en sortie est situé dans un espace au niveau d'un emplacement qui correspond à l'emplacement au niveau duquel l'image est affichée au niveau du deuxième verre (103) de manière à attirer le deuxième œil de l'utilisateur sur l'image affichée.

2. Appareil selon la revendication 1, dans lequel le processeur est configuré pour amener les données, qui indiquent comme quoi le deuxième œil (204) de l'utilisateur est déterminé comme étant dirigé au niveau de l'image affichée, à être stockées en mémoire.

3. Appareil selon la revendication 1 ou la revendication 2, agencé de telle sorte que le deuxième verre (103) affiche une image comportant une pluralité d'objets, l'un de la pluralité d'objets étant désigné comme étant un objet cible ; et
le processeur est configuré pour déterminer si le deuxième œil (204) de l'utilisateur est dirigé au niveau de l'objet cible.

4. Appareil selon la revendication 3, agencé de telle sorte que l'objet cible est affiché avec une indication visuelle, l'indication visuelle indiquant que le deuxième œil (204) de l'utilisateur devrait être dirigé au niveau de l'objet cible.

5. Appareil selon l'une quelconque des revendications 1 à 4, agencé de telle sorte que le deuxième verre (103) affiche une image en mouvement ; et
le processeur est configuré pour déterminer si le deuxième œil (204) de l'utilisateur est en mesure de suivre l'image en mouvement.

6. Appareil selon l'une quelconque des revendications 1 à 5, comportant un projecteur configuré pour projeter une image sur le deuxième verre (103) de manière à fournir une image à des fins de visualisation par le deuxième œil (204) de l'utilisateur.

7. Appareil selon l'une quelconque des revendications 1 à 6, dans lequel le premier verre (102) est configuré pour se fermer de manière sélective par l'affichage d'aucune image ou d'une image noire.

8. Appareil selon l'une quelconque des revendications 1 à 7, dans lequel le processeur est configuré pour déterminer, en fonction de l'entrée reçue, si le deuxième œil (204) de l'utilisateur est dirigé au niveau de l'image affichée pendant une période de temps qui dépasse une période de temps de seuil.

9. Appareil selon l'une quelconque des revendications 1 à 8, dans lequel :
le deuxième verre (103) est agencé pour afficher différentes images à différents moments ; et
le processeur est configuré pour :
recevoir une entrée en provenance du capteur (105) pour chaque image affichée ;
déterminer si le deuxième œil (204) de l'utilisateur est dirigé au niveau de chacune des images affichées ; et
amener les données, qui indiquent si le deuxième œil (204) de l'utilisateur est déterminé comme étant dirigé au niveau de chacune des images affichées, à être stockées en mémoire.

10. Appareil selon l'une quelconque des revendications 1 à 9, comportant un émetteur servant à connecter l'appareil à un dispositif client séparé au moyen d'une technologie sans fil et dans lequel l'émetteur sert à émettre au dispositif client les données qui indiquent si le deuxième œil de l'utilisateur est déterminé comme étant dirigé au niveau de l'image affiché.

11. Appareil selon l'une quelconque des revendications 1 à 9, dans lequel le premier verre et le deuxième verre (103) sont tous les deux en mesure d'afficher une image à des fins de visualisation par un œil correspondant de l'utilisateur et sont en mesure d'être fermés de manière sélective de façon à empêcher la lumière d'atteindre un œil correspondant de l'utilisateur.
